Europäisches Patentamt

European Patent Office

Office européen des brevets

(19)

(11) Veröffentlichungsnummer: **0 327 805**
**A2**

## (12) EUROPÄISCHE PATENTANMELDUNG

(21) Anmeldenummer: 89100218.0

(51) Int. Cl.⁴: **A61K 6/04**

(22) Anmeldetag: 07.01.89

(30) Priorität: 10.02.88 DE 3804022

(43) Veröffentlichungstag der Anmeldung:
16.08.89 Patentblatt 89/33

(84) Benannte Vertragsstaaten:
AT BE CH DE ES FR GB GR IT LI LU NL SE

(71) Anmelder: Degussa Aktiengesellschaft
Weissfrauenstrasse 9
D-6000 Frankfurt am Main 1(DE)

(72) Erfinder: Diehl, Walter, Dr. Dipl.-Phys.
Gustav-Adolf-Strasse 29
D-6450 Hanau 1(DE)
Erfinder: Ringelstein, Hans-Martin
Inheidener Strasse 22
D-6000 Frankfurt(DE)

(54) Zahnfüllmaterial und Verfahren zu seiner Herstellung.

(57) Zahnfüllmaterial mit Härten größer 100 HV für Stopfgoldanwendungen erhält man aus Goldlegierungspulver, das als Legierungsbestandteile Metalle enthält, die zu aushärtbaren Goldlegierungen führen und gleichzeitig salpetersäurelöslich sind. Durch Behandlung mit Salpetersäure erhält man dann eine reine Goldschicht auf den Pulverteilchen.

EP 0 327 805 A2

## Zahnfüllmaterial und Verfahren zu seiner Herstellung

Die Erfindung betrifft ein Zahnfüllmaterial aus einem Goldlegierungspulver und gegebenenfalls einem organischen Bindemittel zum Einbringen in Zahnkavitäten mit Hilfe von Goldstopfverfahren und ein Verfahren zur Herstellung dieses Zahnfüllmaterials.

Die Füllung von Zahnkavitäten mit sogenanntem Stopfgold ist eine der ältesten Zahnfüllungsmethoden. Dazu wird chmisch reines Gold in Form von Goldfolie, Goldschwamm oder Goldpulver in die Kavität eingebracht und mechanisch verfestigt. Stopfgold besitzt im kompakten Zustand eine Härte von rund 25HV, die beim Verarbeiten durch eine zusätzliche Kaltverfestigung auf 30 bis 50 HV gesteigert werden kann. Durch die Art der Verarbeitung unterliegen diese Werte jedoch starken örtlichen Schwankungen, hervorgerufen durch die angewandte Stopftechnik und die verschiedenen Stopfzeiten. Außerdem erfolgt durch die Temperaturbelastungen im Mund ein langsamer Abfall der Kaltverfestigung. Klassische Stopfgoldfüllungen sind daher für mit starker Belastung verbundene Indikationen ungeeignet.

Aus der DE-OS 34 03 779 ist ein Zahnfüllmaterial für Goldstopfverfahren bekannt, das aus plättchenförmigem Goldpulver und einem bei 20 bis 45°C sich verflüssigenden, plastischen organischen Bindemittel, insbesondere Polyäthylenglykol, besteht und durch Einwirkung von Ultraschall verfestigt wird. Die hierbei erreichbaren Härten liegen allerdings auch nur im Bereich von 40 HV. Die generelle Verwendung von Goldlegierungen für Stopfgold ist aus der DE-OS 30 42 008 bekannt.

Es war daher Aufgabe der vorliegenden Erfindung, ein Zahnfüllmaterial aus einem Goldlegierungspulver und gegebenenfalls einem organischen Bindemittel zum Einbringen in Zahnkavitäten mit Hilfe von Stopfgoldverfahren zu entwickeln, mit dem Härten von mehr als 100 HV erreichbar sind. Außerdem sollte ein geeignetes Verfahren zur Herstellung eines solchen Zahnfüllmaterials gefunden werden.

Diese Aufgabe wird erfindungsgemäß dadurch gelöst, daß das Goldlegierungspulver als Legierungsbestandteile Metalle enthält, die zu aushärtbaren Goldlegierungen führen und gleichzeitig salpetersäurelöslich sind, und daß die Oberflächenschicht des Goldlegierungspulvers aus reinem Gold besteht und aufgerauht ist.

Vorzugsweise enthält das Goldlegierungspulver als Legierungsbestandteile insgesamt bis zu 50 % eines oder mehrere Edelmetalle aus der Gruppe Palladium und Silber und eines oder mehrere der Unedelmetalle Zink, Kupfer, Indium und Gallium.

Die Schichtdicke der Goldschicht auf den Gold-legierungspulververteilchen liegt vorteilhafterweise zwischen 0,1 und 2 μm, insbesondere zwischen 0,5 und 1,5 μm.

Außerdem kann das Goldlegierungspulver mit einem zwischen 20 und 45°C sich verflüssigenden, organischen Bindemittel in eine pastöse Konsistenz gebracht werden.

Die Herstellung dieses Zahnfüllmaterials erfolgt in der Weise, daß man Goldlegierungspulver entsprechender Zusammensetzung durch eine Temperaturbehandlung aushärtet und anschließend auf dem Pulver durch Einwirkung von Salpetersäure eine legierungsmetallfreie, aufgerauhte Oberflächenschicht aus reinem Gold erzeugt.

Die Behandlung in Salpetersäure, vorzugsweise bei höherer Temperatur, erfolgt solange, bis sich eine legierungsmetallfreie Goldschicht von 0,1 bis 2 μm Dicke gebildet hat.

Das so erhaltene Goldpulver verbindet die Härte der ausgehärteten Goldlegierungspulververteilchen mit der leichten Verschweißbarkeit von reinem Gold. Es sind auf diese Weise Härten zwischen 100 und 120 HV erreichbar, so daß dieses Zahnfüllmaterial auch für Indikation mit starker Belastbarkeit verwendbar ist.

Als Legierungsbestandteile sind alle Metalle verwendbar, die zu einer aushärtbaren Goldlegierung führen. Bei Dentalgoldlegierungen sind eine ganze Reihe solcher Metallkombinationen bekannt. Vorzugsweise verwendet man Goldlegierungen, die Palladium und gegebenenfalls Silber als Hauptlegierungsbestandteil und zusätzliche ein Unedelmetall aus der Reihe Zink, Kupfer, Indium und Gallium, einzeln oder zu mehreren, enthalten.

Folgende Beispiele sollen die Herstellung und Zusammensetzung solcher Zahnfüllmaterialien näher erläutern:

1. Eine Goldlegierung mit 35 % Silber, 10 % Palladium und 5 % Zink wurde in einer Naßverdüsungsanlage pulverisiert, die Kornfraktion ≤ 45 μm ausgesiebt und in einer Kugelmühle unter Alkohol 1 Stunde lang gemahlen. Man erhält so ein plättchenförmiges Pulver, das zum Aushärten 15 Minuten bei 800°C unter Schutzgas getempert, abgeschreckt und anschließend 15 Minuten bei 400°C angelassen wurde. Anschließend folgte eine sechstündige Behandlung des Pulvers in kochender Salpetersäure, wodurch eine legierungsmetallfreie Oberflächenschicht von etwa 1μm auf den Pulverteilchen entstand. Nach dem Auswaschen und Trocknen lag ein schwer rieselndes, hochaktives Pulver vor, das in einer Modellkavität verdichtet wurde und einen Härtewert von 115 HV erreichte.

2. Eine Goldlegierung mit 18,5 % Silber, 11 % Palladium und 3,5 % Indium wurde analog Beispiel 1 verarbeitet. Die Härte der Versuchsfüllung betrug 105 HV.

3. Eine Goldlegierung mit 25 % Silber, 8 % Palladium, 11,5 % Kupfer und 0,5 % Zink wurde analog Beispiel 1 verarbeitet. Man erhält eine Füllung mit einer Härte von 110 HV.

## Ansprüche

1. Zahnfüllmaterial aus einem Goldlegierungspulver und gegebenenfalls einem organischen Bindemittel zum Einbringen in Zahnkavitäten mit Hilfe von Goldstopfverfahren,
dadurch gekennzeichnet,
daß das Goldlegierungspulver als Legierungsbestandteile Metalle enthält, die zu aushärtbaren Goldlegierungen führen und gleichzeitig salpetersäurelöslich sind, und daß die Oberflächenschicht des Goldlegierungspulvers aus reinem Gold besteht und aufgerauht ist.

2. Zahnfüllmaterial nach Anspruch 1,
dadurch gekennzeichnet,
daß das Goldlegierungspulver insgesamt bis zu 50 % eines oder mehrere Edelmetalle aus der Gruppe Palladium und Silber und eines oder mehrere der Unedelmetalle Zink, Kupfer, Indium und Gallium enthält.

3. Zahnfüllmaterial nach Anspruch 1 und 2,
dadurch gekennzeichnet,
daß die Schichtdicke der Goldschicht auf dem Goldlegierungspulver 0,1 bis 2 $\mu$m beträgt.

4. Verfahren zur Herstellung von Zahnfüllmaterial nach Anspruch 1 bis 3,
dadurch gekennzeichnet,
daß Goldlegierungspulver entsprechender Zusammensetzung durch eine Temperaturbehandlung ausgehärtet und anschließend auf dem Pulver durch Einwirkung von Salpetersäure eine legierungsmetallfreie, aufgerauhte Oberflächenschicht aus reinem Gold erzeugt wird.